# EUROPÄISCHE PATENTSCHRIFT

(11) **EP 1 812 107 B1**
(45) Veröffentlichungstag und Bekanntmachung des Hinweises auf die Patenterteilung: **24.06.2009**
(21) Anmeldenummer: 05815863.5
(22) Anmeldetag: 11.11.2005
(51) Int. Cl.: A61N 1/362

(54) **VORRICHTUNG ZUM VORHERSAGEN VON TACHYARRHYTHMIEN UND / ODER VORHOFARRYTHMIEN**
DEVICE FOR PREDICTING TACHYARRHYTHMIAS AND/OR ATRIAL ARRHYTHMIAS
DISPOSITIF DE PREDICTION DE TACHYARYTHMIES ET/OU D'ARYTHMIES VESTIBULAIRES

(30) Priorität: 12.11.2004 DE 102004054751
(43) Veröffentlichungstag der Anmeldung: 01.08.2007
(73) Patentinhaber: Schirdewan, Alexander, 12555 Berlin (DE); Wessel, Niels, 13156 Berlin (DE)
(72) Erfinder: Schirdewan, Alexander, 12555 Berlin (DE); Wessel, Niels, 13156 Berlin (DE)
(74) Vertreter: Baumbach, Friedrich
(86) Internationale Anmeldenummer: PCT/DE2005/002040
(87) Internationale Veröffentlichungsnummer: WO 2006/050717

(56) Entgegenhaltungen:
- WO-A-98/32489
- US-A- 6 128 534
- US-A- 6 144 878
- US-B1- 6 238 422

## Beschreibung

Die Erfindung bezieht sich auf eine Vorrichtung zum Vorhersagen von Tachyarrhythmien und / oder Vorhofarrhythmien sowie ein elektrisches Therapiegerät mit einer solchen Vorrichtung.

### Hintergrund der Erfindung

Derartige Vorrichtungen sind üblicherweise als elektrische Therapiegeräte, insbesondere Stimulatoren wie implantierbare Herzschrittmacher, Kardioverter oder Defibrillatoren ausgeführt. Ein grundlegendes Wirkprinzip dieser Vorrichtungen besteht darin, elektrische Signale des Herzens zu erfassen und in Abhängigkeit von den erfaßten elektrischen Signalen elektrische Impulse an das Herz auszugeben.

Erste implantierte Kardioverter-Defibrillatoren (ICD) wurden vor etwa 40 Jahren konzipiert. Die erste Implantation im Tierversuch erfolgte 1976, beim Menschen 1980. Der erste programmierbare Defibrillator wurde 1988 implantiert, 1991 erstmals mit endokardialen Elektroden. Seit 1994 werden die stark volumenreduzierten Geräte subpectoral implantiert. Das System des ICD besteht aus einem Generator und entsprechendem Elektrodensystem. Dieses System überwacht ständig die elektrische Aktivität des Herzens. Beim Eintreten von ventrikulären Tachyarrhythmien kann das Gerät die jeweils programmierte elektrische Therapie abgeben: eine hochenergetische Defibrillation, eine niedrigenergetische Kardioversion oder antitachykarde Stimulation. Das Gerät umfaßt einen hermetisch versiegeltes Titangehäuse und einen eingegossenen Kopf, in dem sich die Anschlüsse für die Elektrodensysteme befinden. Energiequellen und Speicherkondensatoren sind in dem Gehäuse untergebracht, in Verbindung mit der Elektronik. Die Verbindung vom Generator zum Patienten wird mittels durch technisch unterschiedlicher Elektrodensysteme hergestellt. Das Elektrogramm-Signal wird zur Auswertung an den Generator übertragen. Kammerarrhythmien können so erkannt und mit der vorher programmierten Therapie behandelt werden. Die zur Verfügung stehende Batteriekapazität erlaubt üblicherweise eine Lebensdauer von mehreren Jahren, danach muß der Generator operativ gegen ein neues Gerät ausgetauscht werden. Neuere Geräte verfügen über ein Datenspeicher zur Aufzeichnung von Patientendaten, Therapieabgaben und Therapieaufzeichnungen, zum Beispiel gespeicherte Elektrogramme, Schlag-zu-Schlag-Intervalle.

Eine Vorrichtung zur Vorhersage von Tachyarrhythmien ist beispielsweise in dem Dokument EP 1 302 158 A2 beschrieben. Die bekannte Vorrichtung kann als implantierbares Therapiegerät ausgeführt werden. Es ist in üblicher Weise ausgebildet, um elektrische Signale des Herzens zu erfassen und elektrische Impulse an das Herz auszugeben. Eine entsprechende Erfassungseinheit für die elektrischen Signale ist dazu mit einer in einen Ventrikel und/oder ein Atrium des Herzens führenden Elektrodenleitung verbindbar. Eine solche Elektrodenleitung kann in bekannter Weise für die uni-, bi- oder multipolare Aufnahme elektrischer Signale aus dem Herzen und eine entsprechende Abgabe von therapeutischen Strom- oder Reaktionsimpulsen an das Herz ausgebildet sein. Für die Aufnahme elektrischer Signale des Herzens sind beispielsweise bipolare Elektrodenleitungen geeignet, welche zusammen mit der Erfassungseinheit so geschaltet werden können, daß die erfaßten Signale uni- oder bipolar aufgenommen werden.

Für die Abgabe von Stimlulations- oder Defibrillationsimpulsen an ein Herz umfaßt ein derartiges Therapiegerät üblicherweise eine Stimulationseinheit. Erfassungs- und Stimulationseinheit eines solchen Implantates sind üblicherweise über eine Steuerung miteinander verbunden, die die Abgabe von Impulsen an das Herz in Abhängigkeit der vom Herzen aufgenommenen elektrischen Größen und gegebenenfalls weiterer Größen steuert. Solche weiteren Größen können im Falle eines ratenadaptiven Herzschrittmachers für den physiologischen Bedarf eines Patienten charakteristisch sein. Als Meßparameter, auf den die Auslösung von Stimulations- oder Defibrillationsimpulsen an das Herz veranlaßt wird, nutzt die bekannte Vorrichtung einen Anstieg des Herzratenvariabilitätswertes.

Des weiteren sind aus dem Dokument US 6,144,878 ein Verfahren und eine Vorrichtung zum Evaluieren der Herzratenvariabilität des Herzens zur Vorhersage von Tachyarrhythmien bekannt. Auch dieses bekannte Verfahren und die bekannte Vorrichtung nutzen zur Vorhersage von Tachyarrhythmien Abweichungen einer gemessenen Herzratenvariabilität von einer als normal definierten Herzratenvariabilität.

### Zusammenfassung der Erfindung

Aufgabe der Erfindung ist es, eine Vorrichtung zur Vorhersage von Tachyarrhythmien und / oder Vorhofarrhythmien zur Verfügung zu stellen, bei der die Genauigkeit und die Zuverlässigkeit der Vorhersage verbessert sind.

Die Aufgabe wird durch eine Vorrichtung zum Vorhersagen von Tachyarrhythmien und / oder Vorhofarrhythmien mit den Merkmalen des Anspruchs 1 gelöst. Nach einem weiteren Aspekt der Erfindung ist ein elektrisches Therapiegerät, insbesondere Stimulator, wie implantierbarer Herzschrittmacher, Kardioverter oder Defibrillator, mit einer derart ausgebildeten Vorrichtung geschaffen. Eine Vorteilhafte Ausgestaltung der Erfindung ist Gegenstand des Unteransprüchs 2.

Erfindungsgemäß ist eine Vorrichtung zum Vorhersagen von Tachyarrhythmien und / oder Vorhofarrhythmien mit den Merkmalen des Anspruchs 1 geschaffen.

Die Erfmdung liegt die Erkenntnis zugrunde, daß der Verlauf des Herzrhythmus im Vorfeld von lebensbedrohlichen Rhythmusstörungen sich in Abhängigkeit vom Auftreten der Rhythmusstörung zu verschiedenen Tageszeiten unterscheidet. Hiervon ausgehend ist die Vorrichtung so ausgebildet, daß die Tageszeitabhängigkeit für die Vorhersage von Tachyarrhythmien und / oder Vorhofarrhythmien berücksichtigt wird. Tageszeitabhängige Normaldaten werden mit aktuellen Meßwerten für Herzsignale verglichen. Die Berücksichtigung der Tageszeitabhängigkeit bei der Vorhersage ermöglicht eine genauere und für den jeweiligen Probanden individuell angepaßte Prognose über das mögliche Auftreten von ventrikulären Tachyarrhythmien und / oder Vorhofarrhythmien.

Als ausgewertete Parameter zur Vorhersage von Tachyarrhythmien und / oder Vorhofarrhythmien werden bevorzugt aktuelle Meßwerte und vorab gespeicherte Normalwerte für die Herzfrequenz (HR) und / oder die Herzfrequenzvariabilität (HRV) herangezogen. Hierbei kann vorgesehen sein, daß die gespeicherten Daten für die zum Vergleich mit den aktuellen Meßwerten herangezogenen Normalwerte einmalig oder in vorgegebenen Zeitabständen mehrmals aktualisiert werden, wobei eine Aktualisierung den Ersatz der vorher gespeicherten Normaldaten durch aktualisierte Normaldaten oder die Berücksichtigung aktueller Normaldaten bedeuten kann, beispielsweise indem vorher gespeicherte und aktuelle Normaldaten gemittelt werden.

Auslösemechanismen von Tachyarrhythmien sind abhängig vom zirkadianen Rhythmus. Die Messung der zirkadianen Herzfrequenz und / oder deren Variabilität dient als Grundlage der Erstellung eines zirkadianen individuellen Profils und der Feststellung der Überschreitung von individuell festgelegten Normwerten für die Vorhersage von Tachyarrhythmien und eine mögliche prophylaktische Differentialtherapie, um eine drohende Kammerarrhythmie zu unterdrücken.

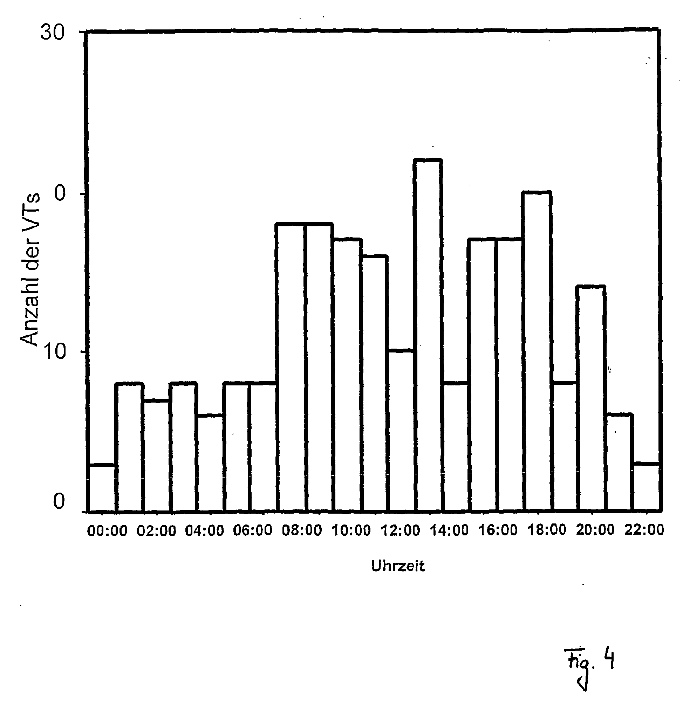

Weiterhin ist Vorhofflimmern eine bedeutsame Rhythmusstörung, die in den Herzvorhöfen generiert wird. Die Herzfrequenzvariabilität (HRV) ist zirkadian-abhängig und vor einem Vorhofflimmern verändert, wodurch eine Vorhersage von Vorhofflimmern ermöglicht werden kann. Die zirkadiane Verteilung des Auftretens von Vorhofflimmern zeigt ein Maximum in der Nacht, d.h. ein gegenläufiges Verhalten zu Kammerrhythmusstörungen (gegenläufig zu Fig. 4 unten).

Das zirkadiane HRV- Profil kann sowohl für die Vorhersage von Kammerarrhythmien als auch von Vorhofflimmern genutzt werden. Ein solches Profil kann mittels Messung der Herzfrequenz und / oder ihrer Variabilität aus den R-Zacken erfolgen (V-Welle im Pacer / ICD - Kammersonde) aber auch aus der Messung der P-Wellen (A-Welle im Pacer / ICD - Vorhofsonde). Die Variabilität der atrioventrikulären Leitung (Vorhof-Kammerkopplung, PR-Intervall, AV-Intervall im Pacer / ICD - Vorhof und Kammersonde) gibt eine zusätzliche zirkadiane Information zusammen mit HR / HRV oder allein zur Vorhersage von Vorhofflimmern und / oder Kammerarrhythmien.

Messung der zirkadianen Vorhoffrequenz und / oder ihrer Variabilität sowie der zirkadianen Vorhof-Kammerkoppelung (PR-Intervall im EKG, AV-Intervall im Schrittmacher / ICD) und / oder deren Variabilität können als Grundlage der Erstellung eines zirkadianen individuellen Profils für die Vorhersage von Vorhofarrhythmien (Vorhofflimmern) und eine mögliche prophylaktische Differentialtherapie um ein drohendes Vorhofereignis zu unterdrücke.

Mit der beschriebenen Vorrichtung ist ein Verfahren zur Vorhersage von Tachyarrhythmien und /oder Vorhofarrhythmien ausführbar.

### Beschreibung von bevorzugten Ausführungsbeispielen

Die Erfindung wird im folgenden anhand von Ausführungsbeispielen unter Bezugnahme auf die Figuren der Zeichnung näher erläutert. Hierbei zeigen:
- Fig. 1: eine schematische Blockdarstellung einer Vorrichtung zur Vorhersage von Tachy- arrhythmien und / oder Vorhofarrhythmien;
- Fig. 2: eine graphische Darstellung eines zirkadianen Rhythmus einer mittleren Herzfre- quenz vor lebensbedrohlichen Rhythmusstörungen;
- Fig. 3: eine graphische Darstellung der mittleren Herzfrequenzdynamik vor lebensbedroh- lichen Rhythmusstörungen zu verschiedenen Tageszeiten;
- Fig. 4: eine graphische Darstellung der zirkadianen Verteilung des Auftretens lebensbe- drohlicher Herzrhythmusstörungen für unterschiedliche Tageszeiten; und
- Fig. 5: eine schematische Darstellung eines zirkadianen Profils für die Herzfrequenz (HR) und die Herzfrequenzvariabilität (HRV) zu verschieden Tageszeiten.

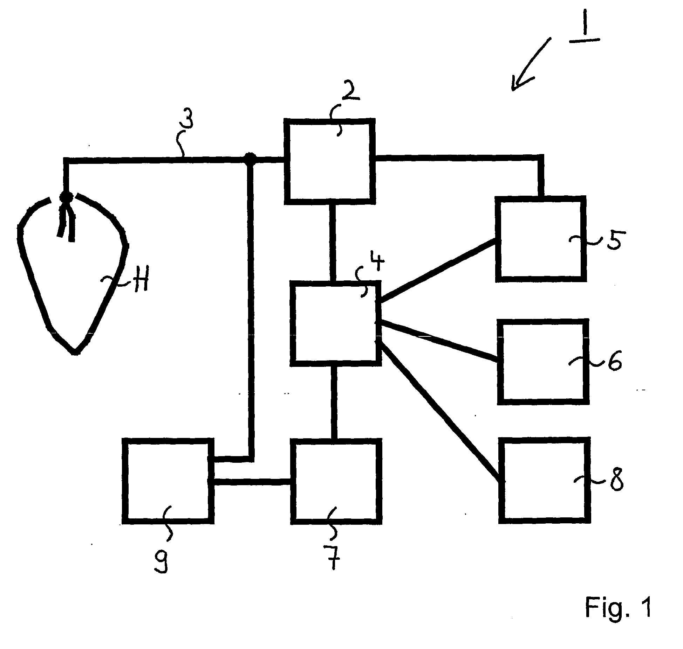

Fig. 1 zeigt eine schematische Darstellung zur Erläuterung einer Vorrichtung 1 zum Vorhersagen von Tachyarrhythmien und / oder Vorhofarrhythmien, bei der es sich beispielsweise um einen implantierbaren Kardioverter/Defibrillator (ICD) handelt. Erfassungsmittel 2 zum Erfassen von elektrischen Meßsignalen eines Herzens H sind mit einer Elektrodenleitung 3 verbunden, die beispielsweise in den rechten Ventrikel des Herzens reicht. Die Erfassungsmittel 1 verfügen über die üblichen Mittel zum Aufnehmen eines intrakardialen Elektrokardiogramms wie beispielsweise einen Eingangsverstärker, sowie einen QRS-Detektor, um den QRS-Komplex in dem intrakardial aufgenommenen Elektrokardiogramm zu detektieren.

Mit Hilfe der Erfassungsmittel 2 kann die jeweils aktuelle Herzfrequenz/Herzrate (HR) aus dem Abstand einer R-Zacke des QRS-Komplexes zur R-Zacke des nächstfolgenden QRS-Komplexes bestimmt werden. Die Bestimmung der Herzfrequenz kann wahlweise mit Hilfe der Erfassungsmittel 2 oder in einer Auswerteeinheit 4 erfolgen. Ausgehend von der Herzfrequenz kann entweder mit Hilfe der Erfassungsmittel 2 oder der Auswertemittel 4 darüber hinaus die Herzfrequenzvariabilität/Herzratenvariabilität (HRV) ermittelt werden, beispielsweise indem die Standardabweichung für die gemessene Herzfrequenz bestimmt wird.

Die in Fig. 1 gezeigte Vorrichtung 1 zur Vorhersage von Tachyarrhythmien und / oder Vorhofarrhythmien verfügt weiterhin über Zeitmittel 5, die mit den Erfassungsmitteln 2 und den Auswertemitteln 4 verbunden sind. Mit Hilfe der Zeitmittel 5 werden zirkadiane elektrische Zeitsignale erzeugt, die in den Erfassungsmitteln 2 und/oder den Auswertemitteln 4 den erfaßten Herzfrequenz-Signalen und/oder den hieraus abgeleiteten Herzfrequenzvariabilitäts-Signalen entsprechend des jeweiligen Erfassungszeitpunktes zugeordnet werden. Auf diese Weise entstehen Wertepaare, die jeweils den Tageszeitpunkt der Meßwerterfassung und den zugehörigen Meßwert für die Herzfrequenz bzw. die Herzfrequenzvariabilität umfassen.

In den Auswertemitteln 4 werden diese erzeugten Meßwertpaare mit zirkadianen Normaldaten für die Herzfrequenz und / oder die Herzfrequenzvariabilität verglichen, die ihrerseits in elektronischen Speichermitteln 6 abgelegt sind. Die Speichermittel 6 sind zur Übertragung der zirkadianen Normaldaten mit den Auswertemitteln 4 verbunden. Wenn bei dem Vergleich der aktuellen Meßwerte mit den zirkadianen Normalwerten eine Überschreitung vorgegebener Schwellwerte festgestellt wird, so wird mit Hilfe der Auswertemittel 4 ein elektrisches Reaktionssignal erzeugt und über mit den Auswertemitteln 4 verbundene Ausgabemittel 7 ausgegeben. Bei den Ausgabemitteln 7 handelt es sich im einfachsten Fall um einen Ausgang zur Abgabe elektrischer Signale der Auswertemittel 4. Vorgegebene Schwellwerte werden bei der Vorrichtung 1 nach Fig. 1 in Schwellwertmitteln 8 gespeichert, die an die Auswertemittel 4 gekoppelt sind. Alternativ kann auch vorgesehen sein, daß die Speichermittel 6 und die Schwellwerhnittel 8 als ein gemeinsamer Speicher ausgeführt sind, indem sowohl die zirkadianen Normaldaten als auch Schwellwertdaten gespeichert sind.

Das elektrische Reaktionssignal wird über die Ausgabemittel 7 an Stimulierungsmittel 9 gegeben, die ihrerseits mit der Elektrodenleitung 3 verbunden sind. In Abhängigkeit von dem elektrischen Reaktionssignal erzeugen die Stimulierungsmittel 9 beispielsweise ein elektrisches Stimulationssignal, um auf festgestellte Rhythmusstörungen des Herzens H zu reagieren.

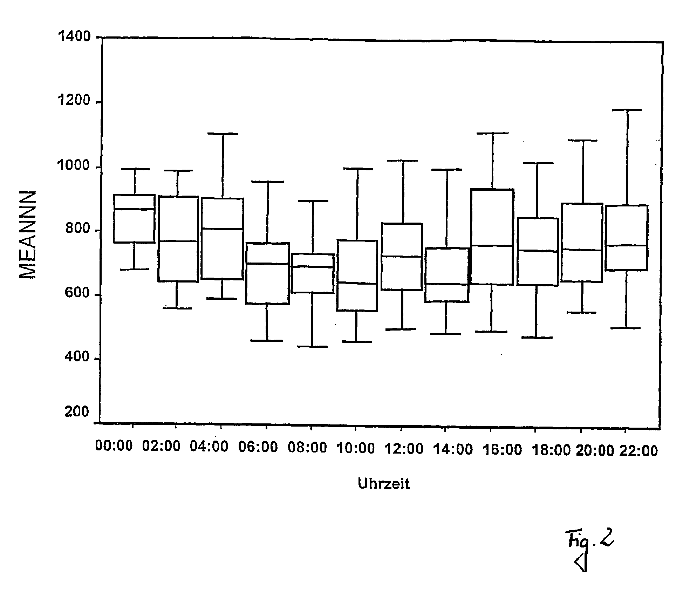

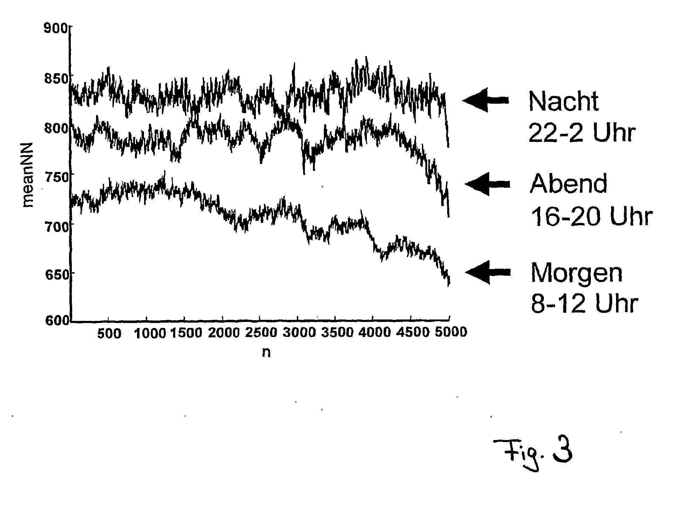

Fig. 2 zeigt eine graphische Darstellung eines zirkadianen Rhythmus der mittleren Herzfrequenz vor lebensbedrohlichen Rhythmusstörungen. Es ist das mittlere Schlag-zu-Schlag-Intervall, welches reziprok zur Herzfrequenz ist, in Abhängigkeit von der Tageszeit dargestellt. Der graphischen Darstellung ist zu entnehmen, daß am Tage eine höhere Herzfrequenz festzustellen ist. Die Herzfrequenzdynamik folgt somit der zirkadianen Rhythmik. Weiterhin ist nachts kein Anstieg der Herzfrequenz vor lebensbedrohlichen Rhythmusstörungen feststellbar, während dieser jedoch abends zehn Minuten und morgens eine Stunde vorher auftritt, wie sich aus Fig. 3 ergibt, die die mittlere Herzfrequenzdynamik (5000 Werte) vor lebensbedrohlichen Rhythmusstörungen morgens, abends und nachts zeigt. Es sind wieder mittlere Schlag-zu-Schlag-Intervalle dargestellt.

Überraschend wurde somit festgestellt, daß sich lebensbedrohliche Rhythmusstörungen zu unterschiedlichen Tageszeiten in unterschiedlicher Art und Weise ankündigen. Darüber hinaus existiert eine zirkadiane Verteilung des Auftretens derartiger Rhythmusstörungen, wie dies Fig. 4 zeigt. In Fig. 4 ist die Häufigkeit des Auftretens von Tachyarrhythmien in Abhängigkeit von der Tageszeit dargestellt.

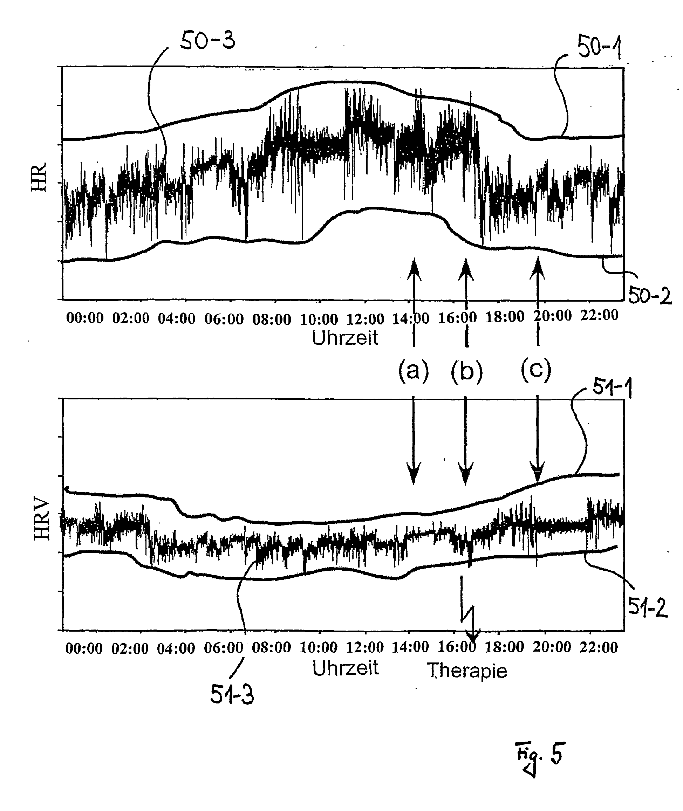

Fig. 5 zeigt eine schematische Darstellung eines zirkadianen Profils für die Herzfrequenz (HR) und die Herzfrequenzvariabilität (HRV) zu verschiedenen Tageszeiten. Während die Kurven 50-1 und 50-2 obere bzw. untere Normal-/Schwellwertdaten für die Herzfrequenz darstellen, zeigt die Kurve 50-3 aktuelle erfaßte Meßwerte der Herzfrequenz. In analoger Weise bilden die Kurven 51-1 und 51-2 zirkadiane Normal-/Schwellwertdaten für die Herzfrequenzvariabilität, wohingegen die Kurve 51-3 aktuellen Meßwerten für die Herzfrequenzvariabilität entspricht. Zum Zeitpunkt (a) wird der obere Schwellwert (Kurve 50-1) für die Herzfrequenz überschritten, es erfolgt jedoch keine Erzeugung eines elektrischen Reaktionssignals, da der aktuelle Meßwert für die Herzfrequenzvariabilität (Kurve 51-3) im Normbereich liegt. Im Gegensatz dazu wird zum Zeitpunkt (b) ein elektrisches Reaktionssignal mit den Auswertemitteln 4 (vgl. Fig. 1) erzeugt, da zusätzlich zu dem aktuellen Meßwert für die Herzfrequenz (Kurve 50-3) auch der aktuelle Meßwert für die Herzfrequenzvariabilität (Kurve 51-3) den unteren Schwellwert (Kurve 51-2) unterschreitet. Bei dem hier dargestellten Ausführungsbeispiel wird zum Zeitpunkt (c) ebenfalls kein elektrisches Reaktionssignal erzeugt, da zu diesem Zeitpunkt der aktuelle Meßwert für die Herzfrequenz (Kurve 50-3) im Normbereich liegt, wohingegen der aktuelle Meßwert für die Herzfrequenzvariabilität (Kurve 51-3) den unteren Schwellwert unterschritten hat.

Die mit der Berücksichtigung der zirkadianen Abhängigkeit des Herzrhythmus bei der Vorhersage von Tachyarrhythmien verbundenen Vorteile ergeben sich auch, wenn mit Hilfe der in Fig. 1 schematisch dargestellten Vorrichtung 1 ein elektronisches Reaktionssignal in den Auswertemitteln 4 erzeugt wird, sobald im Unterschied zu dem in Fig. 5 dargestellten Ausführungsbeispiel entweder nur der aktuelle Meßwert für die Herzfrequenz (Kurve 50-3) oder der aktuelle Meßwert für die Herzfrequenzvariabilität (Kurve 51-3 in Fig. 5) außerhalb des jeweiligen Normbereiches liegt. In beiden Fällen wird jedoch die zirkadiane Abhängigkeit berücksichtigt.

## Patentansprüche

1. Vorrichtung zum Vorhersagen von Tachyarrhythmien und / oder Vorhofarrhythmien mit elektrischen Herzsignale eines Herzens erfassenden Erfassungsmitteln (1) und die erfassten elektrischen Herzsignale auswertenden Auswertemitteln (4), wobei die Erfassungsmittel (1) an die Auswertemittel (4) gekoppelt sind, mit
- zirkadiane Zeitsignale liefernden Zeitmittellen (5), wobei zugehörige zirkadiane Zeitsignale von Herzfrequenz-Signalen und von Herzfrequenz variabilitäts-Signalen die aus den erfassten elektrischen Herzsignalen abgeleitet sind, zugeordnet werden **gekennzeichnet durch**,
- an die Auswertemittel (4) gekoppelte Speichermittel (6) mit darin gespeicherten individuellen oberen und unteren schwellwertkurven für die normale Herzfrequenz und die normale Herzfrequenzvariabilität als Funktion der zirkadianen Zeit.
- an die Auswertemittel (4) gekoppelte und elektrische Reaktionssignale ausgebende Ausgabemittel (7),
und
- die Reaktionssignale von den Auswertemitteln (4) als Reaktion auf einen Vergleich der individuellen Schwellwertkurven mit den erfassen Herzfrequenz-und Herzfrequenzvariabilitäts-Signalen unter Berücksichtigung der zugehörigen zirkadianen Zeitsignale erzeugt und an die Ausgabemittel (7) übergeben werden,
- wobei eine Reaktion erfolgt, wenn die Schwellwertkurven für die Herzfrequenz und oder für die Herzfrequenzvariabilität über- oder unterschritten werden.

2. Elektrisches Therapiegerät, insbesondere Stimulator wie implantierbarer Herzschrittmacher, Kardioverter oder Defibrillator, **gekennzeichnet durch** eine Vorrichtung nach Ansprüch 1.

## Claims

1. Device for forecasting of tachyarrhythmias and / or atrial arrhythmias with electrical means of detecting cardiac signals of a heart (1) and means of evaluation evaluating the electrical cardiac signals detected (4), in which context the means of detection (1) have been coupled to the means of evaluation (4), with
- means of time providing circadian time signals (5), in which context matching circadian time signals are allocated from heart rate signals and from heart rate variability signals which have been derived from the detected electrical cardiac signals, wherein there exist
- means of storage (6) coupled to the means of evaluation (4) with individual upper and lower threshold value curves for the normal heart rate and the normal heart rate variability as a function of the circadian time stored therein,
- output means (7) connected to the means of evaluation (4) and emitting electrical reaction signals,
and
- reaction signals from the means of evaluation (4) are generated as a reaction to a comparison of the individual threshold value curves with the detected heart rate and heart rate variability signals, taking the matching circadian time signals into due account, and are transmitted to the means of output (7),
- in which context there is a reaction if the threshold value curves for the normal heart rate and/or the normal heart rate variability are exceeded or fallen short of.

2. Electrical therapy device, in particular stimulator such as implantable pacemaker, cardioverter or defibrillator, wherein there exists a device according to Claim 1.

## Revendications

1. Dispositif destiné à pronostiquer les tachyarythmies et / ou les arythmies par fibrillations auriculaires avec des moyens de détection (1) enregistrant les signaux électriques d'un coeur et avec des moyens d'évaluation (4) évaluant les signaux électriques du coeur enregistrés, les moyens de détection (1) et les moyens d'évaluation (4) étant jumelés, avec
- des moyens de mesure du temps fournissant des signaux de temps circadiens (5), les signaux de temps circadiens afférents étant attribués à des signaux de fréquence cardiaque et à des signaux de variabilité de la fréquence cardiaque qui dérivent des signaux électriques du coeur enregistrés, **se caractérisant par**
- des moyens de mémorisation (6) jumelés aux moyens d'évaluation (4) avec des courbes de valeurs seuils supérieures et inférieures individuelles pour la fréquence cardiaque normale et la variabilité normale de la fréquence cardiaque qui y sont mémorisées en tant que fonction du temps circadien,
- des moyens de sortie (7) jumelés aux moyens d'évaluation (4) et émettant des signaux électriques de réaction,
et
- les signaux de réaction des moyens d'évaluation (4) sont générés en tant que réaction à une comparaison des courbes de valeurs seuils individuelles avec les signaux de la fréquence cardiaque et de la variabilité de la fréquence cardiaque enregistrés, en tenant compte des signaux de mesure du temps circadiens afférents et sont transmis aux moyens de sortie (7),
- une réaction se produisant quand les courbes de valeurs seuils pour la fréquence cardiaque et/ou la variabilité de la fréquence cardiaque sont dépassées vers le haut ou vers le bas.

2. Appareil électrique de traitement, en particulier stimulateur tel qu'un pacemaker implantable, cardioverter ou défibrillateur, **se caractérisant par** un dispositif conformément à la revendication 1.
